Europäisches Patentamt

European Patent Office

Office européen des brevets

(19)

(11) Veröffentlichungsnummer: **0 114 440**
**B1**

(12) **EUROPÄISCHE PATENTSCHRIFT**

(45) Veröffentlichungstag der Patentschrift:
**29.10.86**

(51) Int. Cl.⁴: **A 61 F 5/47**

(21) Anmeldenummer: **83201803.0**

(22) Anmeldetag: **16.12.83**

(54) **Okklusivpessar.**

(30) Priorität: **20.01.83 CH 323/83**

(43) Veröffentlichungstag der Anmeldung:
**01.08.84 Patentblatt 84/31**

(45) Bekanntmachung des Hinweises auf die Patenterteilung:
**29.10.86 Patentblatt 86/44**

(84) Benannte Vertragsstaaten:
**AT BE CH DE FR GB IT LI LU NL SE**

(56) Entgegenhaltungen:
**EP - A - 0 070 068**
**FR - A - 2 253 537**
**GB - A - 1 308 823**
**US - A - 3 683 905**
**US - A - 3 779 241**

(73) Patentinhaber: **Cimber, Hugo, Dr. med.,**
**Neufeldstrasse 134, CH-3012 Bern (CH)**

(72) Erfinder: **Cimber, Hugo, Dr. med., Neufeldstrasse 134,**
**CH-3012 Bern (CH)**

(74) Vertreter: **Bovard, Fritz Albert et al, Bovard AG**
**Patentanwälte VSP Optingenstrasse 16,**
**CH-3000 Bern 25 (CH)**

ACTORUM AG

## Beschreibung

Es sind Okklusivpessare bekannt, die als ein- oder mehrteilige, starre Körper ausgebildet sind. Diese lassen sich nur mühsam einführen und wieder entfernen und schliessen das Risiko einer Verletzung der Schleimhaut bzw. des Eingrabens in die uterine Wand nicht aus. Um insbesondere diese Nachteile bis zu einem gewissen Grade zu vermeiden, wurden auch schon IUDs (intra uterine devices) mit voneinander wegfedernden Armen vorgeschlagen, welche Arme mit je einem Endkörper versehen waren, dessen gewölbte Aussenflächen dazu dienten, das Verletzungsrisiko der Schleimhaut etwas herabzusetzen, und andererseits in zusammengelegter Stellung ein Ovoid zu bilden, ohne dass aber natürlich diese Endkörper dazu bestimmt und geeignet waren, irgendeine Dichtungsfunktion auszuüben. Eine gewisse kontrazeptive Wirkung beruhte bei diesen IUDs, die übrigens oft noch mit einem Kupferdraht umwickelt waren, auf einer Reizung der Schleimhaut durch den eingeführten Fremdkörper. Schliesslich sind Pessare bekannt (EP-A-0 070 068), welche ein mittels einer entsprechenden Zuleitung aufblasbares Abschlussorgan aufweisen. Je nach der Ausgestaltung solcher Pessare mit aufblasbaren Verschlussorganen können diese sehr wirkungsvoll sein, weil, wenn beispielsweise die Verschlussorgane als aufblasbare kugelige Körper mit sehr dünner Wandung ausgebildet sind, diese Abschlussorgane ohne Reizung der Gebärmutterschleimhaut und ohne sich in die Schleimhaut einzugraben einen absolut dichten Verschluss der Eileitermündungen erlauben, da diese Verschlussorgane dank dem geschilderten Aufbau sehr weich ausgestaltet werden können. Des weiteren erlauben solche Pessare den freien Ablauf des Menstruationsblutes.

So vorteilhaft solche Pessare gegebenenfalls sein können, so bleibt deren Herstellung doch höchst problematisch, und wenn sie überhaupt gelingt, so sind solche Pessare derart kostspielig, dass sie keine allgemeine Verbreitung finden können. Ferner liegt der Nachteil ausschliesslich aus aufblasbarem, weichem Material bestehender Okklusivpessare darin, dass diese die Aufgabe des Verschlusses der Eileitermündung in den Uterus nicht unbedingt erfüllen können, da sie sich in den meisten Fällen nicht wunschgemäss ausspreizen, und infolge der Ausbildung der Äste (3 bzw. 3a) keine genügende Gewähr dafür besteht, dass die Abschlussorgane an die richtige Stelle gebracht werden können.

Der vorliegenden Erfindung liegt die Aufgabe zugrunde, einen Okklusivpessar gemäss dem Oberbegriff des Patentanspruches 1 zu schaffen, der die erwähnten Nachteile dadurch vermeidet, dass die Abschlussorgane als Vollkörper ausgebildet sind und damit einen noch sichereren Abschluss der Eileitermündung gestatten und dazuhin in flexibler Weise am freien Ende des betreffenden Astes angebracht sind. Diese Flexibilität der Verbindung zwischen einem Ast und dem betreffenden Abschlussorgan gestattet, diese Anpresskörper auch dann in die richtige Stellung zu bringen, wenn allein durch die Ausspreizung der Äste eine genauere Positionierung der Abschlussorgane nicht erreicht werden kann. Des weiteren gestattet die erfindungsgemässe Ausbildung nach dem kennzeichnenden Teil des Patentanspruches 1 einen sicheren Abschluss der Eileitermündung, und zwar ohne Verhinderung des Abflusses des Menstruationsblutes. Schliesslich lässt sich ein solcher Okklusivpessar auf vergleichsweise einfache Art herstellen.

In der Zeichnung ist eine beispielsweise Ausführungsform des Erfindungsgegenstandes dargestellt, und zwar

Fig. 1 in teilweisem Schnitt bei strichpunktiert angedeuteter Gebärmutter.

Fig. 2 zeigt einen Schnitt nach der Linie II–II in Fig. 1, während in

Fig. 3 bei geschnittenem Einführröhrchen die Lage der beiden Äste des in das Röhrchen zurückgezogenen Pessars ersichtlich ist.

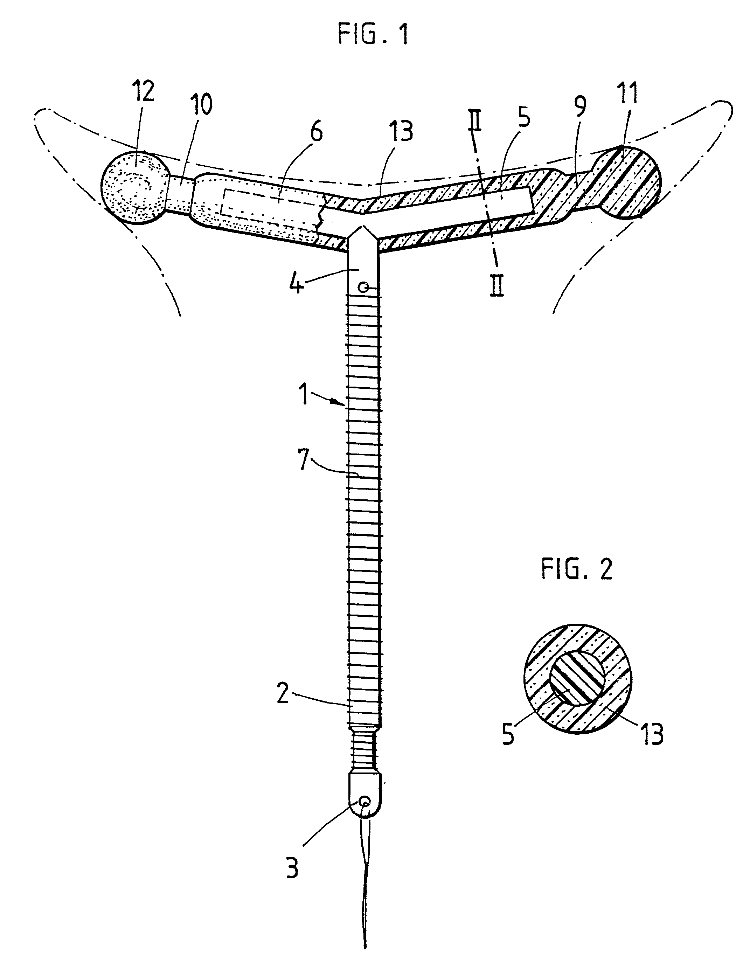

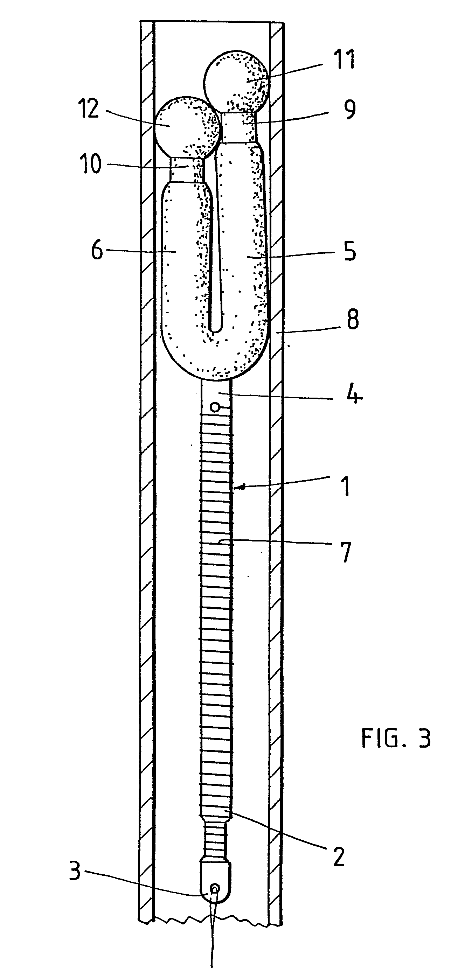

Beim dargestellten Ausführungsbeispiel weist der Pessar einen Tragbalken 1 auf, dessen eines, in der Einführrichtung hinteres Ende 2 mit einer Öse 3 für das Durchschlaufen eines Ausziehfadens versehen ist, während an seinem anderen, in der Einführrichtung vorderen Ende 4 zwei aus dem gleichen Material bestehende Äste 5 und 6 angeordnet sind. Der Tragbalken 1 kann, wie in Fig. 1 dargestellt, mit einem Kupferfaden 7 umwickelt sein, dessen empfängnisverhütende Wirkung ganz allgemein bekannt ist. Das Material des Tragbalkens 1 und der Äste 5 und 6 ist ein steifer, aber weitgehend elastischer Kunststoff, so dass, wenn beispielsweise der Pessar in ein Röhrchen 8 eingeführt wird, wie dies in Fig. 3 dargestellt ist, und man ihn aus der dargestellten Stellung so weit nach vorne (in der Zeichnung oben) vorstösst, bis der Anlenkpunkt der beiden unterschiedlich langen Äste 5 und 6 das obere Ende des Röhrchens 8 verlassen hat, die beiden Äste 5 und 6 sich seitlich voneinander wegspreizen und dabei in eine Stellung gelangen, wie sie in Fig. 1 dargestellt ist.

Die beiden Äste 5 und 6 sind von einem äusserst weichen, elastischen Material, beispielsweise Silikongummi (z.B. dem unter dem geschützten Namen «SILASTIC 382» der Firma Dow Corning verkauften Produkt) ummantelt, welche Ummantelung 13 in Fortsetzung der freien Enden der Arme in zum Zwecke der Erhöhung der Flexibilität angeordnete, an den freien Enden der Äste 5 und 6 anschliessende Verbindungsstücke 9 und 10 geringeren Durchmessers übergeht, an welchen dann schliesslich die im wesentlichen kugeligen und aus dem betreffenden weichen Material bestehenden Abschlussorgane 11 und 12 angeordnet sind.

Der dargestellte Okklusivpessar wird von einer Stellung ausgehend, wie sie in Fig. 3 dargestellt ist, vermittels des Röhrchens 8 in die Gebärmutter eingeführt, wobei eine nicht dargestellte Stange dazu dient, den Pessar innerhalb des Röhrchens 8 vor-, beziehungsweise aus dem Röhrchen 8 hinauszuschieben. Aus der dargestellten Lage des Pessars innerhalb des Einführungsröhrchens 8 ergibt sich, dass infolge der unterschiedlichen Länge der beiden Äste 5 und 6 in aufgeklappter Stellung das kugelige Abschlussorgan 12 des Astes 6 gegen das Verbindungsstück 9 des Astes 5 anliegt, wodurch der Durchmesser des Gesamtpessars entscheidend verringert werden kann. Dies, sowie die Tatsache, dass die Abschlussorgane 11 und 12 aus äusserst weichem Material bestehen, hat zur Folge, dass sowohl die Einführung des Pessars als auch sein Herausziehen schmerzlos erfolgen können.

Sobald der Pessar innerhalb des Einführungsröhrchens 8 weit genug vorgeschoben ist, spreizen sich die Äste 5 und 6 dank der Steifheit ihres Materials in die in Fig. 1 dargestellte Lage, in welcher die Abschlussorgane 11 und 12 schonend und weich im Bereich der Eileitermündungen gegen die Gebärmutterschleimhaut anstehen und damit diese Eileitermündungen dichtend abschliessen. Dank der Weichheit des Materials der Abschlussorgane 11 und 12 sowie der Verbindungsstücke 9, 10 wird eine Anpassung dieser Abschlussorgane 11 und 12 an die besonderen Gegebenheiten der Gebärmutter ermöglicht, und das über das steife Ende jedes Astes 5 resp. 6 hinausstehende und aus einem Verbindungsstück 9 oder 10 und einem Abschlussorgan 11 oder 12 bestehende Teil ist in der Lage, gegebenenfalls auch unter eigener Deformation, den gewünschten Abschluss zu bewerkstelligen.

Das Herausnehmen des Pessars erfolgt wie üblich durch einen durch die Öse 3 durchgeschlauften Faden, indem bei einem Zug auf diesen Faden der Tragbalken 1 und die unter elastischer Deformation zusammenlegbaren Äste 5 und 6 dank ihrer verschiedenen Länge in die in Fig. 3 dargestellte Lage gebracht und so durch den Gebärmutterhals herausgezogen werden können.

Der beschriebene Okklusivpessar gestattet, auf schonendste Weise, d.h. unter Vermeidung jeder Reizung der Gebärmutterschleimhaut und unter Gewährung des freien Abflusses des Menstruationsblutes, einen sicheren Abschluss der Eileitermündungen zu gewährleisten, wobei er ebenso schmerzlos eingeführt wie herausgenommen werden kann. Dazuhin stellt die Herstellung keine weiteren Probleme, so dass der betreffende Pessar einer weiten Verbreitung zuführbar ist.

**Patentansprüche**

1. In die Gebärmutter einzuführender Okklusivpessar, bestehend aus einem Tragbalken (1) aus steifem und elastisch deformierbarem Material, aus zwei vom Tragbalken (1) an seinem einen Ende seitlich abstehenden, mit dem Tragbalken (1) aus einem Stück geformten Ästen (5, 6) und aus je einem am freien Ende jedes Astes (5, 6) angeordneten, kugeligen Abschlussorgan (11, 12) aus weichem, elastisch deformierbarem Material, wobei die Abschlussorgane (11, 12) dazu bestimmt und geeignet sind, ausschliesslich die Mündungen der Eileiter in die Gebärmutter zu verschliessen, dadurch gekennzeichnet, dass die kugeligen Abschlussorgane (11, 12) als Vollkörper ausgebildet und in flexibler Weise am freien Ende des betreffenden Astes (5, 6) angebracht sind.

2. Okklusivpessar nach Patentanspruch 1, dadurch gekennzeichnet, dass die Äste (5, 6) mit dem weichen Material der Abschlussorgane (11, 12) ummantelt sind.

3. Okklusivpessar nach den Patentansprüchen 1 und 2, dadurch gekennzeichnet, dass zwischen den freien Enden der Äste (5, 6) und den Abschlussorganen (11, 12) ein Verbindungsstück (9 bzw. 10) aus leicht deformierbarem, weichem Material angeordnet ist.

4. Okklusivpessar nach Patentanspruch 1, dadurch gekennzeichnet, dass die Äste (5, 6) von unterschiedlicher Länge sind.

**Revendications**

1. Pessaire occlusif destiné à être introduit dans l'utérus, constitué d'un tronc porteur (1) en un matériau déformable élastiquement et rigide, de deux branches (5, 6) s'écartant latéralement du tronc (1), à une de ses extrémités, formées d'une pièce avec le tronc (1) et d'organes obturateurs (11, 12) situés chacun à l'extrémité libre de l'une des dites branches (5, 6), de forme sphérique, en un matériau déformable élastiquement et mou, ces organes obturateurs (11, 12) étant destinés et propres à fermer exclusivement les embouchures des trompes utérines dans l'utérus, caractérisé en ce que les dits organes obturateurs (11, 12) sont des corps pleins, reliés de manière flexible à l'extrémité libre des branches (5, 6).

2. Pessaire occlusif selon la revendication 1, caractérisé en ce que les branches (5, 6) sont enrobées du matériau mou des organes obturateurs (11, 12).

3. Pessaire occlusif selon les revendications 1 et 2, caractérisé en ce qu'un pièce de liaison (9, 10) en un matériau mou, facilement déformable, est disposée entre chacune des extrémités libres des branches (5, 6) et l'organe obturateur (11, 12) correspondant.

4. Pessaire occlusif selon la revendication 1, caractérisé en ce que les branches (5, 6) sont de différentes longueurs.

**Claims**

1. Occlusive pessary to be introduced into the uterus, consisting of a supporting beam (1) of stiff and elastically deformable material, of two branches (5, 6) projecting laterally from the supporting beam (1) at one of its ends and formed in one piece with the supporting beam (1), and of spherical sealing-off parts (11, 12) of soft, elastically deformable material disposed one at the free end of each branch (5, 6), the sealing-off parts (11, 12) being intended and suited for occluding solely the mouths of the Fallopian tubes into the uterus, characterized in that the spherical sealing-off parts (11, 12) are formed as solid bodies and are affixed in a flexible manner to the free ends of the respective branches (5, 6).

2. Occlusive pessary according to patent claim 1, characterized in that the branches (5, 6) are jacketed with the soft material of the sealing-off parts (11, 12).

3. Occlusive pessary according to patent claims 1 and 2, characterized in that between the free ends of the branches (5, 6) and the sealing-off parts (11, 12), a connecting piece (9 and 10, respectively) of slightly deformable, soft material is disposed.

4. Occlusive pessary according to patent claim 1, characterized in that the branches (5, 6) are of differing lengths.

FIG . 1

FIG. 2

FIG. 3